# EUROPÄISCHE PATENTSCHRIFT

(11) **EP 0 570 612 B1**
(45) Veröffentlichungstag und Bekanntmachung des Hinweises auf die Patenterteilung: **13.08.1997**
(21) Anmeldenummer: 92108636.9
(22) Anmeldetag: 21.05.1992
(51) Int. Cl.: A61M 16/00

(54) **Verfahren und Vorrichtung zur Steuerung und unabhängigen Überwachung eines sehr kleinen Glasflusses**
Method and device for controlling and monitoring the flow of a small quantity of gas
Méthode et dispositif pour contrôler et surveiller le débit d'une petite quantité de gaz

(43) Veröffentlichungstag der Anmeldung: 24.11.1993
(73) Patentinhaber: Siemens-Elema AB, 171 95 Solna 1 (SE); SIEMENS AKTIENGESELLSCHAFT, 80333 München (DE)
(72) Erfinder: Olsson, Sven Gunnar, S-232 00 Arlöv (SE); Rydgren, Göran, S-230 44 Bunkeflostrand (SE); Larsson, Anders, S-244 41 Kävlinge (SE)

(56) Entgegenhaltungen:
- EP-A- 0 032 349
- EP-A- 0 039 932
- EP-A- 0 343 542
- FR-A- 2 548 549
- GB-A- 2 053 512
- GB-A- 2 176 313
- Servo-Ventilator-300 , Siemens Broschüre

## Beschreibung

Die Erfindung betrifft ein Verfahren und eine Vorrichtung zur unabhängigen Überwachung der Durchflußmenge und gegebenenfalls der Konzentration eines weiteren Gases gemäß dem Oberbegriff des Patentanspruches 1.

Aus der GB-A-2 176 313 ist ein Gerät zum Steuern verschiedener Gasflüsse mit separaten Ventilen und zum Überwachen der tatsächlichen Gasflüsse mit separaten Flußmessern bekannt. Ein Mikroprozessor berechnet den prozentualen Gasanteil, den Gesamtfluß und die notwendigen Sollwerte für die Ventile. Beim Ändern eines Parameters, z.B. des Gesamtflusses, ändert der Mikroprozessor alle andern Parameter derart, daß das Mischungsverhältnis beibehalten wird. Unter- oder überschreitet ein gemessener Gasflußwert einen Grenzwert, so kann ein Alarm ausgelöst oder auf manuellen Betrieb umgestellt werden. Eine zusätzliche Überwachung eines besonders kleinen Gasflusses ist hierbei jedoch nicht vorgesehen.

Die EP-A-343 542 offenbart ein Gerät zur Atemunterstützung, das zwei separate Regelkreise, einen für Luft und einen für Sauerstoff sowie einen zusätzlichen für den Gesamtatemdruck aufweist. Letzterer beeinflußt die Sollwerte der Luft- und Sauerstoffregler. Die Regelkreise für Luft und Sauerstoff enthalten je einen Gasflußmesser. Zusätzlich ist ein Gesamtgasflußmesser vorhanden, dessen Signal der Summe der Luft- und Sauerstoffgasflußsignale entsprechen muß und der eine Überwachung der Sauerstoffkonzentration ermöglicht, was auch nur möglich ist, wenn beide Gasflüsse relativ groß sind.

Bei der ventilatorkontrollierten oder ventilatorunterstützten Beatmung von Menschen oder Tieren kann es beispielsweise bei niedriger Lungenkapazität von Vorteil sein, dem Atemgas noch ein weiters Gas in sehr niedriger Konzentration beizumischen. So hat sich beispielsweise gezeigt, daß eine Beimischung von Stickstoffoxid (NO) in einer Konzentration von 10-100 ppm die Durchblutung der Alviolen fördert und damit den Gasaustausch in den Lungen steigert.

Weiterhin kann es auch wünschenswert sein, ein nicht am Gasaustausch teilnehmendes Gas wie SF₆ in ähnlich kleinen Konzentrationen dem Atemgas beizumischen, um mit Hilfe dieses Gases beispielsweise das Lungenvolumen messen zu können.

Das sind nur zwei Beispiele dafür, daß es unter gegebenen Umständen wünschenswert sein kann, ein zusätzliches Gas in sehr geringer Konzentration dem Atemgas beizumischen, wobei Atemgas im weitesten Sinne hier alle Gase oder Gasgemische umfassen soll, die bei der Respiratorbehandlung oder Narkose verwendet werden.

Insbesondere bei der Beimischung von Stickstoffoxid (NO) muß berücksichtigt werden, daß dieses Gas bereits in geringen Konzentrationen giftig ist und daß daher die Konzentration in der Größenordnung 10-100 ppm sorfältig überwacht werden muß. Außerdem ist Stickstoffoxid in größeren Konzentrationen zusammen mit O₂ unstabil und kann daher stabil nur stark verdünnt, beispielsweise als 1% Lösung in Stickstoffgas bestehen. Selbst bei einer gewünschten Konzentration des Stickstoffoxids von 100 ppm und einer Verdünnung auf 1 % in Stickstoffgas beträgt das beizumischende Stickstoffoxid/Stickstoffgasgemisch lediglich 1 % des übrigen Atemgases. Der entsprechende Gasfluß liegt innerhalb üblicher Fehlergrenzen für den gesteuerten Gasfluß des Atemgases und ist gegenüber diesem praktisch vernachlässigbar.

Für einige Zusatzgase ließe sich die Konzentration mit Hilfe eines zusätzlichen Gasanalysators bestimmen, was aber einen erheblichen zusätzlichen Aufwand darstellen würde.

Der Erfindung liegt die Aufgabe zugrunde, die Durchflußmenge und die Gaskonzentration des weiteren Gases ohne Gasanalysator und unabhängig von üblicherweise vorhandenen Gasflußregelungen einfach und sicher überwachen zu können. Eine weitere Aufgabe der Erfindung besteht darin, diese Überwachung unabhängig von dem verwendeten weiteren Gas zu machen. Eine weitere Aufgabe der Erfindung besteht darin, die Sicherheit für die mit einem derartigen weiteren Gas beatmeten Lebewesen weiter zu erhöhen.

Diese Aufgaben werden erfindungsgemäß durch die Merkmale der Ansprüche 1 bzw. 21 gelöst. Vorteilhafte Weiterbildungen ergeben sich aus den Unteransprüchen.

Die Erfindung geht dabei von dem Grundkonzept eines bekannten Ventilators, beispielsweise des Servoventilators 300 aus, wie er in der Broschüre "Die inovative Lösung für fortschrittliche Respirator-Therapie", 1991, prinzipiell beschrieben ist. Dieser Ventilator enthält für jedes Gas ein separates Gasmodul, mit dessen Hilfe der Gasfluß für jedes Gas mit hoher Genauigkeit und sehr kurzer Ansprechzeit gesteuert werden kann. Das in jedem Modul enthaltene Ventil mit Druckmesser, Flußmesser und elektronischer Regelung ist beispielsweise in der europäischen Patentanmeldung 90120843.9 (EP-A-483401) beschrieben. Ein Modul kann beispielsweise für die Zufuhr von Luft verwendet werden, ein weiteres für die Zufuhr von reinem Sauerstoff. In jedem Modul wird der Gasfluß über einen Flußmesser bestimmt und zur Steuerung des Ventils herangezogen, um den am Ventilator einstellbaren Sollfluß sicherzustellen. Zusätzlich enthält der Servoventilator 300 einen weiteren Flußmesser in der Exspirationsleitung, um den exspiratorischen Gasfluß zu messen. Dieser exspiratorische Gasflußmesser kann zur Kontrolle des Gesamtflusses, der durch die inspiratorischen Gasventile eingestellt wird, herangezogen werden. Addiert man die Signale, die die Flußmesser der einzelnen Module erzeugen, so entspricht die Summe dieser Signale dem Gesamtfluß und sollte unter der Voraussetzung, daß keine Lecks vorliegen, gemittelt beispielsweise über einen Atemzug dem exspiratorischen Fluß entsprechen.

Zur Zufuhr eines weitern Gases wie beispielsweise Stickstoffoxid kann ein weiteres Modul vorgesehen sein. Unter Umständen ist es notwendig, das Ventil für das weitere Gas zu modifizieren, um so extrem kleine Gasmengen überhaupt mit genügender Genauigkeit steuern zu können. Beispielsweise kann es notwendig sein, die Ventilöffnung zu verkleinern und den Meßbereich der Flußmesser zu verändern.

Auch dieses Ventil ist mit einem eingenen Gasflußmesser versehen. Da der durch dieses Ventil erzeugte Gasfluß aber gegenüber dem Atemgasfluß vernachlässigbar ist, ist eine Kontrolle dieses Ventils und des damit erzeugten Gasflusses über den exspiratorischen Totalgasfluß nicht möglich. Selbst wenn man zunächst davon ausgeht, daß die Servoreglung dieses Ventils mit genügender Genauigkeit den gewünschten und eingestellten Gasfluß erzeugt, kann man sich aus Sicherheitsgründen nicht uneingeschränkt allein darauf verlassen. Erfindungsgemäß ist daher eine unabhängige Überwachung der Gaskonzentration des weiteren Gases vorgesehen. Dazu wird der weitere Gasfluß über einen zusätzlichen Flußmesser, der vorteilhafter Weise unmittelbar hinter dem Ausgang des entsprechenden Steuerventils angeordnet ist, gegebenenfalls mit angepaßtem Meßbereich ein zweitesmal gemessen, das Ausgangssignal dieses Flußmessers wird nun aber nicht mit dem Sollwert für den einzustellenden Fluß verglichen, sondern mit einem Signal, das vom Signal des ersten Flußmessers abgeleitet wird. Das heißt, das Signal des zusätzlichen Flußmessers und damit der Gasfluß des weiteren Gases wird mit dem tatsächlich erzielten Atemgasfluß verglichen, wobei der Atemgasflußwert um einen Faktor reduziert wird, um den der weitere Gasfluß kleiner sein soll. Ist beispielsweise vorgesehen, daß der weitere Gasfluß 1 % des Atemgasflusses entsprechen soll, wird das Ausgangssignales des ersten Flußmessers um den Faktor 100 verkleinert oder das Ausgangssignal des zusätzlichen Flußmessers um den Faktor 100 vergrößert. Alternativ kann auch der Meßbereich des weiteren und/oder zusätzlichen Flußmessers entsprechend geändert sein. Danach werden die Signale verglichen. Bei einer Abweichung über einen vorgebbaren Wert wirdt ein Alarm ausgelöst oder die Durchflußmenge des weiteren Gases beeinflußt. In Weiterbildung der Erfindung wird die Zufuhr des weiteren Gases gestoppt, das heißt das weitere Ventil geschlossen. Wenn das Atemgas aus mehreren Komponenten, beispielsweise Luft und Sauerstoff besteht, wird als Vergleichswert die Summe beider Flußmesser verwendet.

Um bei der Zufuhr von Stickstoffoxid zu verhindern, daß sich unter der Einwirkung des Sauerstoffs im Atemgas NO₂ bildet, sollte die Mischung des weiteren Gases mit dem Atemgas möglichst nahe am Patienten oder sogar erst in dessen Atemwegen erfolgen. Eine Konzentrationsmessung im Gasgemisch wird dabei praktisch unmöglich. Durch das erfindungsgemäße Verfahren und die entsprechende Vorrichtung ist jedoch die Konzentrationsbestimmung und Überwachung vorteilhaft möglich.

In Weiterbildung der Erfindung ist vorgesehen, daß der Sollwert für die Steuerung des ersten Ventils und damit für die Steuerung des Atemgasflussen auch auf ein erstes Potentiometer geschaltet ist und das von diesem Potentiometer einstellbar abgegriffene Signal den Sollwert für das weitere Ventil bildet. Der Sollwert für das weitere Ventil wird damit dadurch gebildet, daß der Sollwert für den Gesamtfluß passiv spannungsgeteilt wird. Es handelt sich hierbei um eine Hardwarelösung. Im Ramen der Erfindung ist es ebenso möglich, die Ableitung des Sollwertes für das weitere Ventil aus dem Sollwerts für den Gesamtgasfluß softwaremäßig auszuführen.

Eine besonders vorteilhafte und einfache Ausgestaltung der Erfindung ergibt sich, wenn ein Doppelpotentiometer vorgesehen ist, wobei das eine Potentiometer wie bereits beschrieben zur Einstellung des Sollwertes für das weitere Ventil dient und auf das andere potentiometer das Ausgangssignal des oder der Flußmesser(s) für das Atemgas geschaltet ist (sind), und daß das dort identisch wie am ersten Potentiometer abgegriffene Signal das vom Atemgasfluß abgeleitete Signal und damit eine Vergleichsgröße für die unabhängige Überwachung der Konzentration des weiteren Gases darstellt.

Konstruktiv vorteilhaft ist es, den zusätzlichen Flußmesser unmittelbar am Ausgang des weiteren Ventils anzuordnen. Dadurch werden zeitliche Verzögerungen, mit denen sich Änderungen des Gasflusses in den Leitungen fortpflanzen, möglichst gering gehalten. Es besteht damit die Möglichkeit, den Gasfluß des weiteren Ventiles ständig mit dem Gasfluß des Atemgases zu vergleichen und damit die Konzentration ständig zu überwachen. Da aber auch hier zeitliche Verzögerung und kurzzeitige Schwankungen nicht ganz ausgeschlossen werden können, ist in Weiterbildung der Erfindung vorgesehen, beide Signale separat über einen Integrator zu integrieren um damit zeitliche Schwankungen herauszumittel. Die Ausgangssignale der Integratoren können über einen Komparator miteinander verglichen werden oder vorteilhaft über zwei Komparatoren, wobei man beispielsweise das vom Istwert des Atemgasflusses abgeleitete Signal derart verstärkt, daß es einen oberen und unteren Grenzwert für die zulässige Konzentration darstellt und das ein Grenzwert den Vergleichswert in einem Komparator und der andere Grenzwert den Vergleichswert in dem anderen Komparator bildet.

Zusätzlich zur Auslösung des Alarmes ist natürlich auch hier bei einer Überschreitung der Grenzwerte die Möglichkeit gegeben, beispielsweise das weitere Ventil vollständig zu schließen, um insbesondere eine überhöhte Konzentration eines Gases wie Stickstoffoxid zu verhindern.

Weitere Ausgestaltungen und Vorteile ergeben sich aus den Ansprüchen.

Im folgenden wird die Erfindung anhand von 4 Figuren näher beschrieben und erläutert. Dabei zeigen:
**Fig.1** schematisch eine Vorrichtung zur Überwachung der Durchflussmenge des weiteren Gases mit kleinem Gasfluß und
**Fig.2** bis 4 drei unterschiedliche Ausführungen der Signalbehandlung zur Erzeugung eines Fehlersignals.

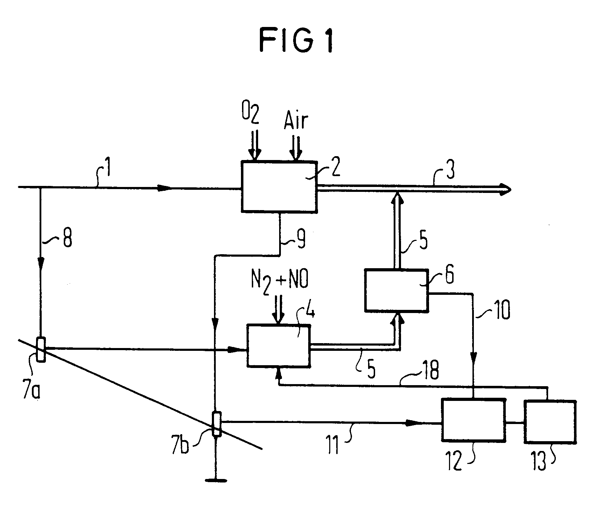

In der Figur 1 ist in einem Blockschaltbild die Steuerung des Atemgasflusses, des weiteren Gasflusses, sowie die unabhängige zusätzliche Überwachung dieses weiteren Gasflusses schematisch dargestellt. Ein Sollwert für den Atemgasfluß wird über eine Leitung 1 auf das oder die Ventile 2 zur Steuerung des Atemgasflusses gegeben. Im vorliegenden Beispiel ist angenommen, daß das Atemgas sich aus Luft und Sauerstoff zusammensetzt, die jeweils über ein separates Ventil gesteuert werden. Der von beiden Ventilen 2 gelieferte Atemgasfluß wird über eine gemeinsame Leitung 3 einem nicht dargestellten Patienten zugeführt. Weiterhin ist ein Ventil 4 für die Steuerung des weiteren Gases, in diesem Falle eines Gasgemisches aus beispielsweise 1 % NO und 99% N₂. Der von dem weiteren Ventil 4 gesteuerte Gasfluß wird über eine Leitung 5 der zum Patienten führenden Leitung 3 zugeführt. Sämtliche Ventile 2,4 sind, was in dem schematischen Blockschaltbild nicht dargestellt ist, mit einem Flußmesser versehen, über den der erzeugte Fluß bestimmt werden kann. In der Leitung 5 vom weiteren Ventil 4 zur Patientenleitung 3 ist ein zusätzlicher Flußmesser 6 vorgesehen. Außerdem ist ein Doppelpotentiometer 7a,7b vorgesehen, über das der Sollwert für das weitere Ventil 4 zur Steuerung der Konzentration von Stickstoffoxid und zur Erzeugung eines Sollwertes für die Überwachung dieser Konzentration abgegriffen werden kann. Auf das Potentiometer 7a ist dazu über eine Leitung 8 der Sollwert für den Atemgasfluß geschaltet, der beispielsweise von der Leitung 1 abgegriffen sein kann. Auf das Potentiometer 7b ist das Ausgangssignal des dem Ventil 2 zugeordneten ersten Flußmessers, der nicht dargestellt ist, über eine Leitung 9 geschaltet. Setzt sich das Atemgas aus Luft und Sauerstoff zusammen und werden beide über separate Ventile gesteuert, so stellt das Signal auf der Leitung 9 die Summe der von zwei Flußmessern kommenden Signale dar.

Weiterhin ist vorgesehen, daß das Signal des weiteren Flußmessers um einen vorgebbaren Faktor, in diesem Falle 100, verstärkt wird, bevor es zur Regelung des Gasflusses des weiteren Gases herangezogen wird. Anstelle dieser Verstärkung kann auch der Meßbereich dieses weiteren Flußmessers entsprechend geändert sein. Das bedeutet, daß der durch dieses Ventil geregelte Gasfluß maximal 1 % des Atemgasflusses beträgt, wenn das Doppelpotentiometer 7a,7b so eingestellt wird, daß der abgegriffene Sollwert dem ursprünglichen Sollwert entspricht. Bei einer anderen Einstellung des Potentiometers 7a,7b beträgt der durch das weitere Ventil 4 gesteuerte Gasfluß nur einen Bruchteil eines %.

Wenn man wie in diesem Beispiel eine geringe Konzentration von Stickstoffoxid dem Atemgas beimischen will, und diese Konzentration in der Größenordnung zwischen 10 und 100 ppm halten will, und wenn darüber hinaus das Stickstoffoxid nur in einer starken Verdünnung in Stickstoffgas stabil ist, so daß eine Mischung von 1 % Stickstoffoxid und 99% Stickstoffgas gewählt wird, so entspricht ein Atemgasfluß durch das weitere Ventil 4 von 1 % des totalen Atemgasflusses einer Konzentration von 100 ppm Stickstoffoxid. Wird über das Potentiometer 7a eine kleinere Spannung abgegriffen und damit ein kleinerer Sollwert für dieses Ventil 4 vorgegeben, so kann eine niedrigere Konzentration erzielt werden. Das weitere Gas wie z.B. NO sollte so weit wie möglich verdünnt werden, um die Durchflußmenge leichter regeln zu können. Gleichzeitig sollte diese Verdünnung aber immer noch so klein sein, daß die Versorgung des Patienten mit Sauerstoff sichergestellt ist. Bei höheren Durchflußmengen des weiteren Gases muß u.U. der Sauerstofffluß oder die Sauerstoffkonzentration angepaßt werden.

Die Regelung des weiteren Ventiles 4 an sich sorgt schon mit einer ausreichenden Genauigkeit für die Einstellung dieser Konzentration. Sie gibt aber keine Garantie dafür, daß diese Konzentration wirklich erzielt wird. Ein Fehler beispielsweise in der Reglung dieses Ventiles 4 oder des weiteren Flußmessers in diesem Ventil kann den weiteren Gasfluß verfälschen und damit die Konzentration, ohne daß normalerweise eine Möglichkeit zur Überwachung gegeben ist. Da die zugeführte Menge an Stickstoffoxid und Stickstoffgas vernachlässigbar klein ist gegenüber dem Atemgas, ist eine Überwachung dieser zugeführten Gasmenge über die Messung des exspiratorischen Gasflusses beispielsweise nicht möglich.

Auf das zweite Potentiometer 7b des Doppelpotentiometers 7a,7b wird das Gesamtsignal der Flußmesser für die Atemgaszufuhr geschaltet und von dort im gleichen Verhältnis abgegriffen wie der Sollwert am ersten Potentiometer 7a. Weiterhin wird das Ausgangssignal des zusätzlichen Flußmessers 6 um den gleichen Faktor verstärkt wie auch das Signal des weiteren Flußmessers, im vorliegenden Fall also um den Faktor 100. Das bedeutet aber, daß das vom zweiten Potentiometer 7b abgegriffene Signal, das somit vom Istwert des Atemgasflußes abgeleitet ist, und das vom zusätzlichen Flußmesser 6 erzeugte Signal nach der Verstärkung um den Faktor 100 gleich groß sein sollten, wenn das System einwandfrei funktioniert. Eine Abweichung dieser Signale voneinander deutet auf einen Fehler hin. In der Figur 1 sind beide Signale über Leitungen 10,11 auf eine Überwachungsanordnung 12 gegeben, in der auf unterschiedlichste Art ein Vergleich der beiden Signale vorgenommen werden und gegebenenfalls ein Alarm ausgelöst werden kann. Zusätzlich kann die Konzentration des weiteren Gases bestimmt und auf eine Anzeige 13 gegeben werden. Im einfachsten Falle können die beiden Signale miteinander direkt verglichen und eine Abweichung über einen vorgegebenen Wert als Kriterium für die Auslösung eines Alarmes verwendet werden. Das Ausgangssignal der Überwachungsanordnung 12 kann über eine weitere Leitung 18 zur Steuerung des Ventils 4 derart verwendet werden, daß beim Vorliegen einer Störung das Ventil 4 geschlossen wird.

Vorteilhafter Weise wird jedoch eine Überwachung vor-geschlagen, wie sie an Hand der folgenden Figuren näher beschrieben ist.

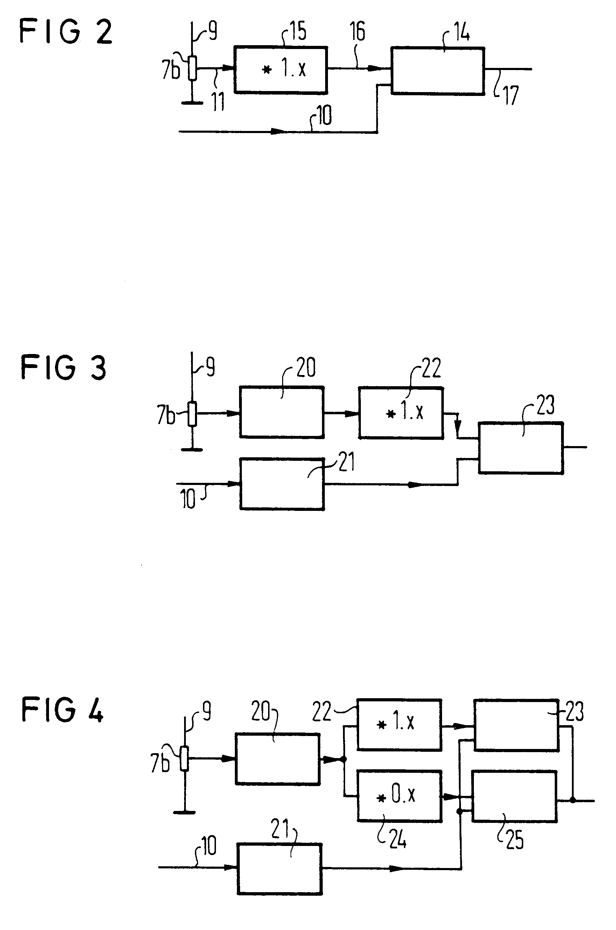

In Figur 2 ist eine erste Ausführungsform der Überwachungsanordnung 12 als Blockschaltbild dargestellt. Sie besteht im wesentlichen aus einem Komparator 14, auf den über die Leitung 10 das Ausgangssignal des zusätzlichen Flußmessers 6 geschaltet ist. Das vom Potentiometer 7b abgegriffene Signal, das den Sollwert für die unabhängige Überwachung des Flusses des weiteren Gases darstellt, ist über die Leitung 11 zunächst auf einen Verstärker 15 und von dort über die Leitung 16 ebenfalls auf den Komparator 14 geschaltet. Bei korrekter Flußregelung sind die Signale auf den Leitungen 10 bzw. 11 gleich groß. Im Verstärker 15 wird der Sollwert geringfügig um einen Faktor 1.x verstärkt und damit eine Grenze für die zulässige Schwankung des Istwertes für den Fluß des weiteren Gases. Überschreitet der Istwert diese Grenze, so wird auf der Ausgangsleitung 17 des Komparators ein Signal erzeugt, das zum Auslösen eines Alarmes verwendet werden kann.

Möglicherweise kann das Signal auf der Leitung 17 noch gefiltert werden, so daß eine Störung erst dann angezeigt wird, wenn das Fehlersignal über einen bestimmten Zeitraum auftritt.

Figur 3 zeigt eine Ausführungsform, bei der Ist- und Sollwert zunächst auf Integratoren 20 bzw.21 geschaltet sind, bevor sie- für den Sollwert wieder über einen Verstärker 22- in einem Komparator 23 verglichen werden. Die Integration kann beispielsweise über einen Atemzug oder auch nur die Inspirationsphase erfolgen. Zeitliche geringfügige Verschiebungen in der Messung der Gasflüsse, die durch unterschiedliche Plazierung der Flußmesser und durch die Trägheit der Gase in den Leitungen hervorgerufen werden, lassen sich damit einfach herausmitteln.

In dem Ausfürungsbeispiel gemäß Figur 4 wird der vom Potentiometer 7b abgegriffene und im Integrator integrierte Wert auf zwei Verstärker 22 und 24 geschaltet, die unterschiedliche Verstärkung haben. So verstärkt der eine z.B. um einen Faktor 1.x und der andere um den Faktor 0.x. Durch diese unterschiedliche Verstärkung lassen sich somit eine obere und eine untere Grenze festlegen, bei deren Über- bzw. Unterschreitung durch den Istwert eine Störmeldung erfolgt.

Die Ausgangssignale der beiden Verstärker 22 und 24 sind dazu auf je einen Komparator 23 bzw. 25 geschaltet, auf die gleichfalls das Ausgangssignal des Integrators 21 geschaltet ist. Der Komparator 22 dient dem Vergleich mit der oberen Grenze, der Komparator 24 dem mit der unteren.

Im Rahmen der Erfindung ist es ebenso möglich, Teile der Überwachungsvorrichtung softwaremäßig auszuführen. So können beispielsweise die Signale auf den Leitungen 8,9 und 10 in Figur 1 digitalisiert und einem Mikroprozessor zugeführt werden, der daraus den Sollwert für das Ventil 4 ableitet und die unabhängige Überwachung des weiteren Gasflusses vornimmt. Auch die Verstärker, Komparatoren und Integratoren können Teil des Mikroprozessors sein. Weiterhin läßt sich mit Hilfe des Mikroprozessors aus den Gasflußwerten für das Atemgas und das weitere Gas, z.B. NO, die Konzentration berechnen und über ein Display anzeigen.

### BEZUGSZEICHENLISTE

- Leitung: 1,3,5,8-11,16-18
- Ventil: 2,4
- Flußmesser: 6
- Potentiometer: 7a,7b
- Überwachungsanordnung: 12
- Anzeige: 13
- Komparator: 14,23,25
- Verstärker: 15,22,24
- Integrator: 20,21

## Patentansprüche

1. Verfahren zur unabhängigen Überwachung der Durchflußmenge und gegebenenfalls der Konzentration eines durch ein regelbares Ventil (4) steuerbaren weiteren Gases zu den Atemwegen eines Menschen oder Tieres, wobei die Größe des Gasflusses des weiteren Gases klein ist gegen die Größe eines Atemgasflusses und von einem vorgebbaren Sollwert abhängt und wobei der Atemgasfluß durch einen ersten Flußmesser (2) und der Gasfluß des weiteren Gases durch einen weiteren Flußmesser gemessen werden, dessen Meßsignal zusammen mit dem vorgebbaren Sollwert zur Steuerung des regelbaren Ventils (4) verwendet wird, **dadurch gekennzeichnet,** daß der Gasfluß des weiteren Gases mit einem zusätzlichen Flußmesser (6) ein zweites Mal gemessen wird und das so erhaltene Meßsignal mit einem von einem den Atemgasfluß repräsentierenden Signal abgeleiteten Signal verglichen und beim Vorliegen vorgebbarer Kriterien ein Alarm ausgelöst oder die Durchflußmenge des weiteren Gases beeinflußt wird.

2. Verfahren nach Anspruch 1, **dadurch gekennzeichnet,** daß der Atemgasfluß durch Spontanatmung erzeugt wird.

3. Verfahren nach Anspruch 1 oder 2, **dadurch gekennzeichnet,** daß das den Atemgasfluß repräsentierende Signal das Ausgangssignal des ersten Flußmessers ist.

4. Verfahren nach Anspruch 1, **dadurch gekennzeichnet,** daß der Atemgasfluß über ein erstes Ventil (2) geregelt wird, auf das zur Steuerung der Größe des Atemgasflusses ein erster Sollwert gegeben wird.

5. Vorrichtung zur Durchführung des Verfahrens nach einem der Ansprüche 1-4 mit einer Vorrichtung (7a) zur Abgabe des vorgebbaren Sollwertes zur Steuerung des regelbaren Ventils (4) und einer Vorrichtung (7b) zur Ableitung eines Signals von einem den Atemgasfluß repräsentierenden Signal, **dadurch gekennzeichnet,** daß das Ausgangssignal des zusätzlichen Flußmessers (6) auf eine Überwachungsanordnung (12) geschaltet ist, die das Signal des Flußmessers (6), gegebenenfalls nach einer Verstärkung um einen vorgebbaren Faktor, mit dem abgeleiteten Signal vergleicht und bei einer Abweichung der beiden Signale voneinander über einen vorgebbaren Wert zumindest einen Alarm auslöst.

6. Vorrichtung nach Anspruch 5, bei der der Atemgasfluß über ein erstes Ventil (2) geregelt wird, **dadurch gekennzeichnet,** daß der zusätzliche Flußmesser (6) zwischen dem Ausgang des regelbaren Ventils (4) und einem Ort, an dem die Gasflüsse beider Ventile (2,4) vereint werden, angeordnet ist.

7. Vorrichtung nach Anspruch 5 oder 6, **dadurch gekennzeichnet,** daß der zusätzliche Flußmesser (6) unmittelbar am Ausgang des regelbaren Ventils (4) angeordnet ist.

8. Vorrichtung nach einem der Ansprüche 5-7, **dadurch gekennzeichnet,** daß bei einer Abweichung der beiden Signale voneinander über einen vorgebbaren Wert das regelbare Ventil (4) geschlossen wird.

9. Vorrichtung nach einem der Ansprüche 5-8, bei der der Atemgasfluß über ein erstes Ventil (2) geregelt wird, auf das zur Steuerung der Größe des Atemgasflusses ein erster Sollwert gegeben wird, **dadurch gekennzeichnet,** daß der Sollwert für die Steuerung des regelbaren Ventils (4) von dem ersten Sollwert abgeleitet ist.

10. Vorrichtung nach Anspruch 9, **dadurch gekennzeichnet,** daß der erste Sollwert auf ein erstes Potentiometer (7a) geschaltet ist und ein davon abgegriffenes und damit einem Teil des ersten Sollwertes entsprechendes Signal den vorgebbaren Sollwert bildet.

11. Vorrichtung nach einem der Ansprüche 5-10, **dadurch gekennzeichnet,** daß das Meßsignal des weiteren Flußmessers um einen vorgegbaren Faktor verstärkt ist, bevor es zur Flußregelung verwendet wird.

12. Vorrichtung nach einem der Ansprüche 5-11, **dadurch gekennzeichnet,** daß der Meßbereich des weiteren und/oder zusätzlichen Flußmessers (6) entsprechend der Größe des zu messenden weiteren Gases eingestellt ist.

13. Vorrichtung nach einem der Ansprüche 5-12, **dadurch gekennzeichnet,** daß das den Atemgasfluß repräsentierende Signal auf ein zweites Potentiometer (7b) geschaltet ist und ein dort abgegriffenes Signal das abgeleitete Signal bildet.

14. Vorrichtung nach den Ansprüchen 10 und 13, **dadurch gekennzeichnet,** daß für das erste und zweite Potentiometer (7a,7b) ein Doppelpotentiometer mit identischen Abgriffen verwendet wird.

15. Vorrichtung nach einem der Ansprüche 5-14, **dadurch gekennzeichnet,** daß das Ausgangssignal des zusätzlichen Flußmessers (6) um einen vorgebbaren Faktor verstärkt ist, bevor es mit dem abgeleiteten Signal verglichen wird.

16. Vorrichtung nach einem der Ansprüche 5-15, **dadurch gekennzeichnet,** daß das abgeleitete Signal auf einen ersten Integrator (20) und das Ausgangssignal des zusätzlichen Flußmessers (6) auf einen zweiten Integrator (21) geschaltet sind und daß die Ausgangssignale beider Integratoren (20,21) auf mindestens einen Komparator (23,25) in der Überwachungsanordnung (12) geschaltet sind, der bei einer vorgebbaren Abweichung der Ausgangssignale der Integratoren (20,21) zumindest einen Alarm auslöst.

17. Vorrichtung nach Anspruch 15, **dadurch gekennzeichnet,** daß zwei Komparatoren (23,25) vorgesehen sind, auf die die beiden Signale der Integratoren (20,21) zumindest teilweise unterschiedlich verstärkt derart geschaltet sind, daß ein Fenster für den zulässigen Gasfluß des weiteren Gases gebildet wird und zumindest ein Alarm beim Überschreiten der oberen oder unteren Grenze des Fensters ausgelöst wird.

## Claims

1. Method for independent monitoring of the flow rate and, if appropriate, the concentration of a further gas which can be regulated by a controllable valve (4) and is delivered to the airways of a human or animal, where the quantity of the gas flow of the further gas is small compared to the quantity of a respiratory gas flow and is dependent on a prescribable set value, and where the respiratory gas flow is measured by a first flow meter (2) and the gas flow of the further gas is measured by a further flow meter whose measurement signal is used, together with the prescribable set value, to regulate the controllable valve (4), characterized in that the gas flow of the further gas is measured a second time with an additional flow meter (6), and the measurement signal (10) thus obtained is compared with a signal (11) derived from a signal representing the respiratory gas flow and, in the presence of prescribable criteria, an alarm is triggered or the flow rate of the further gas is affected.

2. Method according to Claim 1, characterized in that the respiratory gas flow is generated by spontaneous ventilation.

3. Method according to Claim 1 or 2, characterized in that the signal representing the respiratory gas flow is the output signal (9) of the first flow meter.

4. Method according to Claim 1, characterized in that the respiratory gas flow is controlled via a first valve (2) to which, for the purpose of regulating the quantity of the respiratory gas flow, a first set value is transmitted.

5. Apparatus for carrying out the method according to one of Claims 1 - 4, with a device (7a) for issuing the prescribable set value for regulating the controllable valve (4), and a device (7b) for deriving a signal (11) from a signal representing the respiratory gas flow, characterized in that the output signal of the additional flow meter (6) is switched to a monitoring arrangement (12) which compares the signal of the flow meter (6) with the derived signal (11), if appropriate after amplification by a prescribable factor, and, if the two signals deviate from one another beyond a prescribable value, triggers at least one alarm.

6. Apparatus according to Claim 5, in which the respiratory gas flow is controlled via a first valve (2), characterized in that the additional flow meter (6) is arranged between the outlet of the controllable valve (4) and a site at which the gas flows of both valves (2, 4) are combined.

7. Apparatus according to Claim 5 or 6, characterized in that the additional flow meter (6) is arranged directly at the outlet of the controllable valve (4).

8. Apparatus according to one of Claims 5 - 7, characterized in that the controllable valve (4) is closed if the two signals deviate from one another beyond a prescribable value.

9. Apparatus according to one of Claims 5 - 8, in which the respiratory gas flow is controlled via a first valve (2) to which, for the purpose of regulating the quantity of the respiratory gas flow, a first set value is transmitted, characterized in that the set value for regulating the controllable valve (4) is derived from the first set value.

10. Apparatus according to Claim 9, characterized in that the first set value is switched to a first potentiometer (7a), and a signal tapped therefrom, and therefore corresponding to part of the first set value, forms the prescribable set value.

11. Apparatus according to one of Claims 5 - 10, characterized in that the measurement signal of the further flow meter is amplified by a prescribable factor before it is used for controlling the flow.

12. Apparatus according to one of Claims 5 - 11, characterized in that the measurement range of the further and/or additional flow meter (6) is adjusted according to the quantity of the further gas to be measured.

13. Apparatus according to one of Claims 5 - 12, characterized in that the signal representing the respiratory gas flow is switched to a second potentiometer (7b), and a signal tapped there forms the derived signal.

14. Apparatus according to Claims 10 and 13, characterized in that a double potentiometer with identical taps is used for the first and second potentiometers (7a, 7b).

15. Apparatus according to one of Claims 5 - 14, characterized in that the output signal of the additional flow meter (6) is amplified by a prescribable factor before it is compared with the derived signal.

16. Apparatus according to one of Claims 5 - 15, characterized in that the derived signal is switched to a first integrator (20) and the output signal of the additional flow meter (6) is switched to a second integrator (21), and in that the output signals of both integrators (20, 21) are switched to at least one comparator (23, 25) in the monitoring arrangement (12), which comparator triggers at least one alarm if there is a prescribable deviation between the output signals of the integrators (20,21).

17. Apparatus according to Claim 16, characterized in that two comparators (23, 25) are provided, to which the two signals of the integrators (20, 21) are switched, at least partly with different amplification, in such a way that a window for the permissible gas flow of the further gas is formed, and at least one alarm is triggered if the upper limit or lower limit of the window is infringed.

## Revendications

1. Procédé pour contrôler de manière indépendante le débit et éventuellement la concentration d'un gaz en supplément dont on peut se rendre maître par une valve (4) réglable vers les voies respiratoires d'un être humain ou d'un animal, la valeur du flux de gaz supplémentaire étant petite par rapport à la valeur d'un flux de gaz de respiration et dépendant d'une valeur de consigne pouvant être prescrite, et le flux du gaz de respiration pouvant être mesuré par un premier débitmètre (2) et le flux du gaz supplémentaire étant mesuré par un débitmètre supplémentaire, dont on utilise le signal de mesure conjointement avec la valeur de consigne pouvant être prescrite pour commander la valve (4) réglable, caractérisé en ce que l'on mesure le flux du gaz supplémentaire une deuxième fois par un débitmètre (6) supplémentaire, on compare le signal de mesure (10) ainsi obtenu à un signal (11) obtenu à partir d'un signal représentant le flux du gaz de respiration et on déclenche une alarme ou on influence le débit du gaz supplémentaire en présence de certains critères pouvant être prescrits.

2. Procédé suivant la revendication 1 caractérisé en ce que l'on produit le flux de gaz de respiration par respiration spontanée.

3. Procédé suivant la revendication 1 ou 2, caractérisé en ce que le signal représentant le flux de gaz de respiration est le signal (9) de sortie du premier débitmètre.

4. Procédé suivant la revendication 1, caractérisé en ce que l'on règle le flux du gaz de respiration par l'intermédiaire d'une première valve (2) à laquelle on donne, pour commander la valeur du flux du gaz de respiration, une première valeur de consigne.

5. Dispositif pour la mise en oeuvre du procédé suivant l'une des revendications 1 à 4, comportant un dispositif (7a) destiné à la fourniture de la valeur de consigne pouvant être prescrite pour la commande de la valve (4) réglable et un dispositif (7b) pour l'obtention d'un signal (11) à partir d'un signal représentant le flux du gaz de respiration, caractérisé en ce que le signal de sortie du débitmètre (6) supplémentaire est branché à un dispositif (12) de contrôle, qui compare le signal du débitmètre (6), éventuellement après une amplification d'un facteur pouvant être prescrit, au signal (11) obtenu et qui déclenche au moins une alarme si les deux signaux s'écartent l'un de l'autre de plus d'une valeur pouvant être prescrite.

6. Dispositif suivant la revendication 5, dans lequel le flux du gaz de respiration est réglé par une première valve (2), caractérisé en ce que le débitmètre (6) supplémentaire est monté entre la sortie de la valve réglable (4) et un lieu où les flux de gaz des deux valves (2,4) sont réunis.

7. Dispositif suivant la revendication 5 ou 6, caractérisé en ce que le débitmètre (6) supplémentaire est monté directement à la sortie de la valve réglable (4).

8. Dispositif suivant l'une des revendications 5 à 7, caractérisé en ce que la valve (4) réglable est fermée lorsque les deux signaux s'écartent l'un de l'autre de plus d'une valeur pouvant être prescrite.

9. Dispositif suivant l'une des revendications 5 à 8, dans lequel le flux du gaz de respiration est réglé par l'intermédiaire d'une première valve (2), à laquelle une première valeur de consigne peut être donnée pour la commande de la valeur du flux du gaz de respiration, caractérisé en ce que la valeur de consigne pour la commande de la valve (4) réglable est obtenue à partir de la première valeur de consigne.

10. Dispositif suivant la revendication 9, caractérisé en ce que la première valeur de consigne est branchée sur un premier potentiomètre (7a) et un signal prélevé de ce potentiomètre et correspondant ainsi à une partie de la première valeur de consigne forme la valeur de consigne pouvant être prescrite.

11. Dispositif suivant l'une des revendications 5 à 10, caractérisé en ce que le signal de mesure du débitmètre supplémentaire peut être amplifié d'un facteur pouvant être prescrit, avant d'être utilisé pour la régulation du flux.

12. Dispositif suivant l'une des revendications 5 à 11, caractérisé en ce que la zone de mesure du débitmètre supplémentaire (6) est réglée en fonction du débit du gaz supplémentaire à mesurer.

13. Dispositif suivant l'une des revendications 5 à 12, caractérisé en ce que le signal représentant le flux du gaz de respiration est branché sur un second potentiomètre (7b), et un signal prélevé de ce potentiomètre forme le signal obtenu.

14. Dispositif suivant les revendications 10 et 13, caractérisé en ce qu'un potentiomètre double à prises identiques est utilisé pour le premier et le second potentiomètre (7a, 7b).

15. Dispositif suivant l'une des revendications 5 à 14, caractérisé en ce que le signal de sortie du débitmètre (6) supplémentaire est amplifié d'un facteur pouvant être prescrit, avant d'être comparé au signal obtenu.

16. Dispositif suivant l'une des revendications 5 à 15, caractérisé en ce que le signal obtenu est branché à un premier intégrateur (20) et le signal de sortie du débitmètre (6) supplémentaire est branché à un second intégrateur (21) et les signaux de sortie des deux intégrateurs (20, 21) sont branchés sur au moins un comparateur (23, 25) dans le dispositif (12) de contrôle, lequel comparateur déclenche au moins une alarme en cas d'écart, pouvant être prescrit, des signaux de sortie des intégrateurs (20, 21).

17. Dispositif suivant la revendication 16, caractérisé en ce qu'il est prévu deux comparateurs (23, 25), sur lesquels les deux signaux des intégrateurs (20, 21) sont branchés au moins en partie en étant amplifiés de manière différente, de telle sorte qu'une fenêtre pour le flux admissible du gaz supplémentaire soit formé et qu'au moins une alarme soit déclenchée lorsque la limite supérieure ou inférieure de la fenêtre est franchie.
